(19) **Europäisches Patentamt**

**European Patent Office**

**Office européen des brevets**

(11) **EP 2 732 813 A1**

(12) **EUROPEAN PATENT APPLICATION**

(43) Date of publication:
**21.05.2014 Bulletin 2014/21**

(51) Int Cl.:
***A61K 9/70*** *(2006.01)*      ***A61K 47/44*** *(2006.01)*
***A61K 31/485*** *(2006.01)*

(21) Application number: **12007710.2**

(22) Date of filing: **14.11.2012**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(71) Applicant: **Hexal AG
83607 Holzkirchen (DE)**

(72) Inventor: **Kohr, Thomas
83607 Holzkirchen (DE)**

(74) Representative: **Kluschanzoff, Harald
Sandoz International GmbH
Global Intellectual Property
Industriestrasse 25
83607 Holzkirchen (DE)**

(54) **Orodispersible film compositions**

(57)      The present invention relates to an orodispersible film composition comprising a therapeutically effective amount of at least one active pharmaceutical ingredient, a gelling agent, at least one flavoring agent selected from natural citrus fruit derived oil or a mixture of two or more natural citrus fruit derived oils, wherein said composition does not comprise an artificial flavoring agent. The present invention also relates to a method of preparation of said composition, and an orodispersible film comprising said composition. The orodispersible film composition is particularly useful as a medicament.

EP 2 732 813 A1

**Description**

Field of the Invention

The present invention relates to an orodispersible film composition comprising at least one active pharmaceutical ingredient, methods of preparing said composition, and an orodispersible film comprising said composition. The orodispersible film composition is particularly useful as a medicament.

Description of Background Art

Oral administration of an active pharmaceutical ingredient in a single dosage form are complex when the intention is to have the active pharmaceutical ingredient to be absorbed into the body fast and with high patient compliance. Avoiding abuse of tablet-form dosage forms and non-compliance with a prescribed dosage regimen due to refusal by the patient may be solved by administration of an orodispersible film. Said orodispersible film is soluble in the mouth at a particular pH and allows for fast release of the active pharmaceutical ingredient. Further the dosage form cannot be spit out as it tends to adhere to the buccal mucosa and disintegrates fast.

Orodispersible films for co-administration of two or more active pharmaceutical ingredients, antagonists and agonists have been described in the prior art. Co-administration of therapeutic agents is in particular useful is the treatment of patients suffering from narcotic dependence. In WO 2011/017483 a co-administration of narcotics agonist and antagonist has been described. In particular, WO 2011/017483 discloses a combination of opioid agonist buprenorphine and antagonist naloxone.

State of the art formulations of said combination are administered in tablet form and have as such the potential to be abused. The combination may also be formulated as an orally dissolvable film dosage form that provides the desired absorption levels of the agonist and antagonist, while providing an adhesive effect in the mouth, rendering it difficult to remove once placed in the mouth, thereby making abuse of the agonist difficult. By providing the orodispersible film composition comprising at least one active pharmaceutical ingredient it is important that the amount of impurities formed in said composition is minimal during the shelf life of the composition.

WO 2011/017483 discloses film composition comprising more than one active pharmaceutical ingredient. However, the prior art film formulations comprise a large number of excipients such as artificial flavorings to mask bitter or unpleasant taste of the pharmaceutical ingredients, which might influence the formulation of impurities in the composition.

Therefore, there is an unmet need for new orodispersible film composition comprising at least one active pharmaceutical ingredient that are stable against forming impurities over an extended period of time.

Summary of the invention

The object of the present invention was to provide an improved orodispersible film composition comprising at least one active pharmaceutical ingredient as well as a more robust, economical and acceptable method of preparation thereof.

It was also an objective of the present invention to provide pharmaceutical compositions in the form of an orodispersible film with a reduced amount of impurities compared to prior art compositions but without reduced shelf-life of the orodispersible film.

In one aspect the present invention provides an orodispersible film composition comprising of a therapeutically effective amount of at least one active pharmaceutical ingredient, a gelling agent enabling film formation and providing a matrix for incorporation of the active pharmaceutical ingredient(s), and at least one flavoring agent selected from natural citrus fruit derived oil or a mixture of two or more natural citrus fruit derived oils. Said composition does not comprise an artificial flavoring agent such as synthetic flavor oils, synthetic flavoring aromatics.

As used herein, the term "orodispersible film" includes thin films and sheets, in any shape, including rectangular, square, or other desired shape that disintegrate when wetted e.g. upon contact with buccal mucosa of the patient or when administered orally, i.e. put on the tongue or ingested sublingually. The thickness and size of the orodispersible films described herein may be adapted to the oral cavity of the user as well as to the desired time for dissolution.

As used herein the term "active pharmaceutical ingredient" refers to a substance in a pharmaceutical composition that is biologically active. Agonists and antagonists are herein covered by the terms as well.

In an embodiment of the present invention, the active pharmaceutical ingredient is selected from the group consisting of opioids, preferably buprenorphine or pharmaceutically acceptable salts thereof, in particular buprenorphine HCl, without being limited thereto. In the embodiment the formulation further comprises, without being limited thereto, an opioid antagonist, preferably naloxone or pharmaceutically acceptable salts thereof, in particular naloxone HCl. One preferred embodiment comprises combinations of opioid agonist and antagonist, preferably of buprenorphine and naloxone or pharmaceutically acceptable salts or combinations thereof.

As used herein, the term "agonist" refers to a chemical substance that is capable of providing a physiological response or activity in the body of the user.

As used herein, the term "antagonist" refers to any chemical substance that acts within the body of the user to reduce the physiological activity of another chemical substance.

In a preferred embodiment of the present invention, the ratio of buprenorphine HCl and naloxone HCl in the film composition is of between 1:1 and 10:1, preferably between 2:1 and 5:1, more preferably 4:1 or 3.5:1.

In another preferred embodiment the percentage of the at least one active pharmaceutical ingredient in the orodispersible film composition is between 1 % and 50 % by weight (w/w), preferably 5 % to 25 % (w/w), more preferably 20 % (w/w). In this context, the term active pharmaceutical ingredient also covers the combination of two or more pharmaceutical ingredients and also encompasses a combination of antagonist(s) and agonist(s), preferably of buprenorphine and naloxone or pharmaceutically acceptable salts and combinations thereof.

In another embodiment of the orodispersible film composition, the composition comprises a percentage of a flavoring agent, i.e. of natural citrus fruit derived oil, of between 0.01 % and 1 % by weight (w/w), preferably of between 0.05 % and 0.5 % (w/w), more preferably of 0.1 % (w/w).

In one embodiment of the present invention, the citrus fruit derived natural oil is selected from the group consisting of lemon, orange and grapefruit oil, or mixtures thereof.

In one embodiment the orodispersible film composition comprises a percentage of the gelling agent of between 20 % and 60 % by weight (w/w), preferably of 50 % (w/w). The amount of gelling agent further depends on the desired film properties, e. g. film texture and dissolution properties as well as production method and may be adapted, accordingly.

In a preferred embodiment, the gelling agent is selected from the group consisting of hydroxypropylcellulose, hydroxypropylmethylcellulose, methylcellulose, polyethylene glycol, polyethylene oxide and ethyl cellulose. While only one single gelling agent may be incorporated into the composition, the invention also covers embodiments of the film composition incorporating mixtures of two and more gelling agents or of the same gelling agent having different molecular weights. For instance, in some embodiments, the films may include polyethylene oxide 100,000 alone or in combination with a second polymer component polyethylene oxide 200,000, a third polymer component polyethylene oxide 900,000 and a fourth polymer component. The fourth polymer may be another water-soluble polymer such as e.g. Pharmacoat 606 or a gelling agent selected from the above defined group. Suitable water-soluble polymers include, without limitation, any of those provided above.

The molecular weight of the incorporated gelling agent, in particular of polyethylene oxide may be further varied. In some embodiments, high molecular weight polyethylene oxide, such as about 4 million, may be desired to increase mucoadhesion of the film. In some other embodiments, the molecular weight may range from about 100,000 to 900,000. In some embodiments, high molecular weight gelling agents (600,000 to 900,000) may be combined with low molecular weight (100,000 to 300,000) gelling agents, e. g. of polyethylene oxide to form the polymer component of the film composition.

The gelling agent provides a polymeric carrier matrix of the film composition. Preferably, the gelling agent is water-soluble without limiting the invention thereto.

A further embodiment the film composition comprises at least one plasticizer, at least one sweetener, at least one buffering agent and/or at least one dye.

The plasticizer alone or in combination with one or more gelling agent(s) define(s) the conditions of oral dissolution and dispersion of the invention films and film ingredients. Orodispersible films may, without being limited thereto, be classified according to their dissolution parameters into three main classes: fast dissolving, moderate dissolving and slow dissolving. Fast dissolving films e.g. dissolve in about 1 second to about 30 seconds in the mouth. Moderate dissolving films e.g. dissolve in about 1 to about 30 minutes in the mouth, and slow dissolving films e.g. dissolve in more than 30 minutes in the mouth. Fast dissolving films may consist of low molecular weight hydrophilic polymers (i.e., polymers having a molecular weight between about 1,000 to 9,000 or polymers having a molecular weight up to 200,000). In contrast, slow dissolving films may have high molecular weight polymers (i.e. having a molecular weight in the millions). Moderate dissolving films tend to fall in between the fast and slow dissolving films. Moderate dissolving films may dissolve rather quickly, but also have a good level of mucoadhesion. Moderate dissolving films are also flexible, quickly wettable, and are typically non-irritating to the user.

In a preferred embodiment of the present invention, the film forming polymers preferably have a molecular weight between 100,000 and 900,000 and thus fall in the category of moderate dissolving films. Moderate dissolving films provide a quick enough dissolution rate, most preferably of about 1 minute to 5 minutes, while providing an acceptable mucoadhesion level such that the film is not easily removable once it is placed in the oral cavity of the user. The invention is not restricted to the aforesaid embodiment of films and also encompasses fast and slow dissolving films as well as combinations thereof, without being limited to the aforesaid parameters. Beside mucoadhesive films, the present invention also encompasses non-mucoadhesive films, in particular films administered sublingually.

In most preferred embodiment of the present invention the plasticizer is selected from the group consisting of glycerol, propylene glycol, dibutyl sebacate, triacetin, triethyl citrate and isopropyl myristate and mixtures thereof, preferably propylene glycol.

The sweetener is selected from the group consisting of monosaccharides, disaccharides, polysaccharides, especially maltodextrin, sucralose, neotame, alitame, cyclamate, sorbitol, xylitol, saccharin, aspartame, acesulfame, maltitol or mixtures thereof. Other sweeteners may also be used.

The buffering agent is selected from the group consisting of tri-sodium citrate and citric acid monohydrate, or mixtures thereof. Buffering agent in the context of the present invention covers substances that are useful to adapt the pH value of the film composition to enhance or define dissolution when ingested.

In another embodiment the orodispersible film composition further comprises a percentage of the plasticizer between 2 % and 15 % by weight (w/w), preferably 5 % (w/w), a percentage of the sweetener between 0 % and 20 % by weight (w/w), preferably 15 % (w/w), and/or a percentage of a dye being between 0 % and 0.5 % by weight (w/w), preferably between 0.001 % and 0.1 % (w/w), more preferably 0.01 % (w/w).

One embodiment of the invention orodispersible film composition comprises or consists of a therapeutically effective amount of at least one active pharmaceutical ingredient, a plasticizer, a buffer substance, a sweetener, a film former, a dye, and at least one natural oil, in particular a natural oil derived from citrus fruits.

Although a variety of different film former polymers may be used, it is preferred to select polymers that provide mucoadhesive or non- mucoadhesive properties to the film, as well as a desired dissolution and/or disintegration rate. In particular, the time period for which it is desired to maintain the film in contact with the mucosal tissue depends on the type of active pharmaceutical ingredient contained in the composition. Some active pharmaceutical ingredient may only require a few minutes for delivery through the mucosal tissue, whereas other actives may require up to several hours or even longer. Accordingly, in some embodiments, one or more water-soluble polymers, as described above, may be used to form the film.

By selecting a specific dye film compositions may be optically adapted to the flavoring agent used in the composition. Thus e.g. yellow color may enhance the citric taste sensation provided by lemon oil incorporated into the film. The dyes used with the invention films are pharmaceutically and physiologically acceptable dyes. Suitable dyes or coloring agents include food, drug and cosmetic colors (FD&C), drug and cosmetic colors (D&C), or external drug and cosmetic colors (Ext. D&C). These colors are dyes, their corresponding lakes, and certain natural and derived colorants. Lakes are dyes absorbed on aluminum hydroxide. Other examples of coloring agents include known azo dyes, organic or inorganic pigments, or coloring agents of natural origin. Inorganic pigments are preferred, such as the oxides or iron or titanium, these oxides.

In a preferred embodiment of the invention the film composition comprises a therapeutically effective amount of a first active pharmaceutical ingredient, in particular buprenorphine HCl, a therapeutically effective amount of a second active pharmaceutical ingredient, in particular naloxone HCl, acting as antagonist to buprenorphine HCl; a first buffer substance, in particular tri-sodium citrate; a second buffer substance, in particular citric acid monohydrate; a first sweetener, in particular acesulfame K; a second sweetener, in particular maltitol; a first film former, in particular polyethylene oxide 100,000; a second film former, in particular polyethylene oxide 200,000; a third film formers, in particular polyethylene oxide 900,000; a fourth film former, in particular Pharmacoat 606; a dye, in particular dye FD & C Yellow No. 6; and a natural oil, in particular natural lemon oil.

In a further embodiment the composition further comprises a plasticizer, in particular propylene glycol.

With the film composition of the present invention, it was surprisingly found that a significant reduction of impurities compared to film compositions comprising artificial flavoring agents could be achieved. In consequence, omitting artificial and nature identical artificial flavoring agents the shelf life of the present film compositions as well as the stability of active pharmaceutical ingredient could be significantly increased. Due to the exclusive use of natural flavoring agents, patient compliance is further enhanced.

In another embodiment the invention orodispersible film has a percentage of a first active pharmaceutical ingredient, preferably buprenorphine HCl from 3 % to 20 % by weight (w/w), more preferably from 4 % to 20 % (w/w), most preferably from 4.6 % to 18.42 % (w/w), a percentage of the second active pharmaceutical ingredient, preferably naloxone HCl from 0.5 % to 6 % (w/w), more preferably from 1 % to 5 % (w/w), most preferably from 1.19 % to 4.8 % (w/w), a percentage of a plasticizer, preferably of propylene glycol from 1 % to 4 % (w/w), more preferably 2.8 % (w/w), a percentage of a first buffer substance, preferably of tri-sodium citrate from 1 % to 4 % (w/w), more preferably 2.86 % (w/w), a percentage of a second buffer substance, preferably of citric acid monohydrate from 2 % to 8 % (w/w), more preferably 6.32 % (w/w), a percentage of a first sweetener, preferably of acesulfame K is 1 % to 4 % (w/w), more preferably 3.2 % (w/w), a percentage of a second sweetener, preferably of maltitol is 5 % to 15 % (w/w), more preferably 12.86 % (w/w), a percentage of a first film former, preferably of polyethylene oxide 100.000 from 8 % to 14 % (w/w), more preferably 12.86 % (w/w), a percentage of a second film former, preferably of polyethylene oxide 200.000 from 20 % to 30% (w/w), more preferably 28.94 % (w/w), a percentage of a third film former, preferably of polyethylene 900.000 from 1 % to 6 % (w/w), preferably 5.15 % (w/w), a percentage of a fourth film former, preferably of Pharmacoat 606 from 3 % to 10 % (w/w), more preferably 4.51 % (w/w), a percentage of a dye, preferably of dye FD & C Yellow No. 6 from 0.01 % to 0.5 % (w/w), more preferably 0.03 % (w/w), and a percentage of the natural oil, preferably lemon oil from 0.01 % to 1 % (w/w), more preferably 0.1 % (w/w). The present invention is not limited to the above combination of active pharmaceutical ingredient and further excipients.

In particular if the film composition of the present invention includes at least one antagonist, it may be desired to control the release of the antagonist, so as to delay or wholly prevent the release of the antagonist from the film when taken orally. In the film that is to be placed in the oral cavity, it is desired to absorb the agonist buccally, so as to provide rapid integration of the agonist into the body of the user. At the same time, it may be desired to prevent or reduce absorption of any antagonist buccally, thereby allowing the antagonist to be swallowed and destroyed in the stomach. Reducing the absorption of an antagonist may be achieved via physical means, such as by encapsulating the antagonist in a material that blocks absorption. It is however possible, to reduce the absorption of the antagonist by chemical means, such as by controlling the local pH of the film.

A variety of optional components and fillers also may be added to the films. These may include, without limitation: surfactants; plasticizers; polyalcohols; anti-foaming agents, such as silicone-containing compounds, which promote a smoother film surface by releasing oxygen from the film.

Additives may be included in the films. Examples of classes of additives include lubricants, blowing agents, pigments, fillers, bulking agents, release modifiers, adjuvants, flow accelerators, granulating agents, diluents, binders, glidants, adhesives, anti-adherents, acidulants, softeners, resins, demulcents, solvents, surfactants, emulsifiers, elastomers and mixtures thereof. These additives may be added together with the active ingredient(s), before or after.

Useful additives include, for example, gelatin, vegetable proteins such as sunflower protein, soybean proteins, cotton seed proteins, peanut proteins, grape seed proteins, whey proteins, whey protein isolates, blood proteins, egg proteins, acrylated proteins, water-soluble polysaccharides such as alginates, carrageenans, guar gum, agar-agar, xanthan gum, gellan gum, gum arabic and related gums (gum ghatti, gum karaya, gum tragancanth) or pectin.

Further additives may be flow agents and opacifiers, such as the oxides of magnesium aluminum, silicon, titanium, etc. It further may be useful to add silicon dioxide, calcium silicate, or titanium dioxide. These compounds act as flow agents and opacifiers.

Other ingredients include binders which contribute to the ease of formation and general quality of the films. Non-limiting examples of binders include starches, pregelatinized starches, gelatin, polyvinylpyrrolidone, methylcellulose, sodium carboxymethyl cellulose, ethyl cellulose, polyacrylamides, polyvinyl oxoazolidone, and polyvinyl alcohols.

Further potential additives include solubility enhancing agents, such as substances that form inclusion compounds with active components. Such agents may be useful in improving properties of very insoluble and/or unstable actives. In general, these substances are molecules with hydrophobic internal cavities and hydrophilic exteriors. Insoluble and/or instable actives may fit within the hydrophobic cavity, thereby producing an inclusion complex, which is soluble in water. Accordingly, the formation of the inclusion complex permits very insoluble and/or instable actives to be dissolved in water. A particularly desirable example of such agents are cyclodextrins, which are cyclic carbohydrates derived from starch. Other similar substances, however, are considered well within the scope of the present invention.

The film composition further desirably contains a buffer to control the local pH of the film composition. Any desired level of buffer may be incorporated into the film composition so as to provide the desired local pH level. The buffer is preferably provided in an amount sufficient to control the release from the film and/or the absorption into the body of the agonist and the optional antagonist.

The film composition of the present invention may include a therapeutically effective amount of an antagonist, to prevent abuse of the agonist. The film composition is administered to a patient through the oral cavity of the patient. The orodispersible film composition is then allowed to dissolve in the oral cavity of the patient for a sufficient time so as to release the active(s) therein. In some embodiments, the film composition may remain in the oral cavity for at least 30 seconds, and in some embodiments may remain in the oral cavity for at least 1 minute. After the film composition is placed into the oral cavity of the patient, the film preferably becomes sufficiently adhered so as to render its removal difficult. After the film composition has been administered to the patient, the active(s) are sufficiently released from the composition and allowed to take effect on the patient.

In a further embodiment the orodispersible film composition is in the form of a non-mucoadhesive film. Such non-mucoadhesive films do not adhere to the buccal mucosa and may thus be employed for sublingual administration.

Orodispersible film compositions comprise a certain amount of impurities, i.e. derivate of the active pharmaceutical ingredients comprised in the dosage form. Impurities accumulate in the dosage form over storage time. It is difficult to quantify the amount of impurities as they form during storage and accumulate within the product. Amount and composition of the impurities depend on the storage conditions, composition, substances such as gelling agent, flavoring agents etc. uses for manufacturing the film composition and production and drying temperature of the film. Further these impurities are rather complex and analysis thus difficult. It is thus desirable to decrease the percentage of impurities in the product also in view of potential negative adverse reactions due to impurities.

In one embodiment the invention film composition comprises buprenorphine as agonist in combination with naloxone as antagonist. The impurities thus consist of buprenorphine and naloxone derivatives, in particular naloxone-10a-hydroxy and/or naloxone-N-desalkyl. Surprisingly, in the film composition of the present invention comprising exclusively natural oils derived from citrus fruits the percentage of impurities formed of naloxone derivates is significantly lower than in compositions comprising artificial favoring agents.

The same applies to embodiments of the invention film composition comprising buprenorphine as active pharmaceutical ingredient or agonist. The impurities thus comprise naloxone derivatives, in particular naloxone-10a-hydroxy and/or naloxone-N-desalkyl and buprenorphine derivates, in particular buprenorphine-descyclopropylmethyl and/or buprenorphine-dimer.

In one aspect the present invention relates to the orodispersible film composition, wherein the content of impurities of naloxone derivatives and/or buprenorphine derivatives is less than 1 % by weight (w/w). In a preferred embodiment of the present invention the content of impurities of naloxone derivatives, in particular naloxone-10a-hydroxy and/or naloxone-N-desalkyl is less than 1 % (w/w). In a preferred embodiment of the present invention the content of impurities of buprenorphine derivatives, in particular buprenorphine-descyclopropylmethyl and/or buprenorphine-dimer is less than 1% (w/w).

Surprisingly, in the invention film composition comprising exclusively natural oils derived from citrus fruits the percentage of impurities derived from buprenorphine and naloxone derivates is significantly lower than in compositions comprising artificial favoring agents. Determination of overall impurity levels in compositions according to the present invention after 2 months of storage under conditions of permanent and accelerated stability testing showed an up to 25-fold decrease in impurities when compared to prior art compositions comprising artificial flavorings.

Thus the compositions according to the present invention provide an improved long-term stability with significantly lower levels of impurities forming during storage.

In another aspect the film compositions of the present invention may be prepared via any desired process. In a preferred embodiment a method of preparing a orodispersible film composition of the invention comprising the steps of: (i) forming a suspension of a therapeutically effective amount of at least one active pharmaceutical ingredient, (ii) adding a gelling agent to the suspension, (iii) adding a natural oil, derived from citrus fruits, in particular lemon, orange or grapefruit oil or mixtures thereof to the suspension, (iv) forming a film and (v) drying the film.

As the production and drying temperature may significantly affect the amount of impurities present in the film it is desirable to carry out step (v) of drying the film at 20 to 80 degrees Celsius, preferably at 30 to 70 degrees Celsius, more preferably at 40 to 65 degrees Celsius, most preferably at 50 degrees Celsius. This method step in combination with avoiding the use of artificial flavoring agents results in a significant decrease of impurities in the finished products even if analyzed over and extended period of storage of the product.

In another embodiment of the method of invention the drying time is adapted and ranges between 1 and 60 minutes, preferably between 5 and 45 minutes, in particular 10 minutes. Drying time and/or temperature alone or in combination or in combination with the composition of the film have a significant impact on the formation of impurities and derivates of the active pharmaceutical ingredients or agonist/antagonist composition or combination.

In yet another embodiment of the method of invention the drying time is of between 1 and 60 minutes, in particular of between 5 and 45 minutes, preferably of 10 minutes at max 35 degrees Celsius, while a further embodiment has a drying time of between 1 and 60 minutes, in particular of between 5 and 45 minutes, preferably of 10 minutes at max 45 degrees Celsius.

In another embodiment of the method of invention the drying time is between 1 and 60 minutes, in particular of between 5 and 45 minutes, preferably of 10 minutes at max 55 degrees Celsius. In one embodiment of the method of invention the drying time is between 1 and 60 minutes, in particular of between 5 and 45 minutes, preferably of 10 minutes at max 65 degrees Celsius. The invention is not limited to the above temperature and time regimens. It is possible to execute a drying method consisting of several, in particular two or more temperature and time steps or changes. An incremental decrease or increase of drying temperature over drying time is encompassed as well.

In a further embodiment the method of the present invention in step (iii) further substance selected from the group consisting of sweetening agent, plasticizers, buffering agent and/or dye is added to the suspension.

Depending on the nature of the selected components one embodiment of the method of the present invention contemplates that the further substance(s) are added to the suspension before, after or simultaneously with the at least one active pharmaceutical ingredient, the gelling agent and/or the natural oil. This results in a more homogenous dispersal of the substances and thus enables even dissolution of the film and release of the active pharmaceutical ingredient(s).

The film compositions of the present invention may be formed via any desired process readily available to the skilled person. In an embodiment of the method of the present invention the film is formed by casting, molding, extrusion or spraying. In an embodiment the wet composition is e.g. cast into a film and then sufficiently dried to form a self-supporting film composition. The wet composition may be cast into individual dosages, or it may be cast into a sheet, where the sheet is then cut into individual dosages.

In an embodiment the film as manufactured by the method of the invention has a wet film thickness of 400 to 800 microns, preferably of 700 to 750 microns, more preferably 460 to 490 microns and/or a dry film thickness of 100 to 200 microns, preferably of 140 to 180 microns. The thickness of the film controls the dispersion and dissolution time and rate of the film and thus directly affects the availability and release of the active pharmaceutical ingredient.

The invention encompasses the use of the orodispersible film composition as described above as a medicament. Orodispersible films that include one or more agonists or partial agonists may be used for the treatment or prevention of drug addiction. The agonist is selected from chemical substances that are capable of providing a physiological response or activity in the body of the user. In one embodiment of the present invention the films as described may further include one or more antagonists. The antagonist is selected from any chemical substance that acts within the body of the user to reduce the physiological activity of another chemical substance. In some embodiments, an antagonist used herein may act to reduce and/or block the physiological activity of the agonist.

Example

An orodispersible film comprising a combination of buprenorphine HCl and naloxone HCl was prepared which included a ratio of buprenorphine to naloxone of 3.5 : 1. The composition is summarized in Table 1 below.

Table 1. Composition of orodispersible film

| Component | Function | Amount (mg) |
|---|---|---|
| Buprenorphine HCl | Active pharmaceutical ingredient | 8.71 |
| Naloxone HCl | Active pharmaceutical ingredient | 2.45 |
| Tri-Sodium citrate | Buffer | 1.35 |
| Citric acid (monohydrate) | Buffer | 2.99 |
| Acesulfame K | Sweetener | 1.51 |
| Malitol | Sweetener | 6.06 |
| Polyethylene oxide 100,000 | Gelling agent | 6.06 |
| Polyethylene oxide 200,000 | Gelling agent | 13.92 |
| Polyethylene oxide 900,000 | Gelling agent | 2.47 |
| Pharmacoat 606 | Gelling agent | 2.16 |
| Lemon oil | Flavoring agent | 0.057 |
| Dye (FD&C Yellow No. 6) | Dye | 0.010 |

A suspension of the buprenorphine and naloxone was prepared and a plasticizer and gelling agent was added to the suspension. In a further step the lemon oil as flavoring agent, the sweeteners and the dye were added. A film was formed by casting a sheet. This sheet was dried at 65°C for 15 minutes. The sheet was then cut to form the final dosage form having an area size of 2.5 cm$^2$ in the present example.

Films were stored for 0 to 6 months under permanent and accelerated stability testing conditions and the total amount of impurities over reporting level were analyzed by HPLC analysis.

The term accelerated stability testing as used herein refers to storage regimes designed according to WHO guidelines for stability testing of active substances and pharmaceutical products. In one embodiment of the present invention an accelerated stability testing regime was applied wherein the tested compositions were stored for up to 6 months at 30 °C and 65 % relative humidity. In another embodiment of the present invention an accelerated stability testing regime was applied wherein the tested compositions were stored for up to 6 months at 40 °C and 75 % relative humidity. The term permanent stability testing as used herein refers to storage regimes designed according to WHO guidelines for stability testing of active substances and pharmaceutical products. In one embodiment of the present invention a permanent stability testing regime was applied wherein the tested compositions were stored for up to 6 months at 25 °C and 60 % relative humidity.

**Decription of the HPLC method**

The measurement of the impurities was obtained using Dionex Ultimate 3000 and Agilent HP 1200 apparatus. The details on the columns and mobile phase are presented in Table 2.

Table 2. Details on the HPLC method.

| Chromatography parameters | |
|---|---|
| Pre-column | ChromGuard R 10 x 3.0 mm Repl5 (Artikel Nr. Agilent CP28186) |
| Column | XBridge 2.5 $\mu$m |
| Column dimensions | 50 x 3.0 mm |
| Column temperature | 40 °C |
| Temperature autosampler | 10 °C |
| Mobile Phase A | sodium octane-1-sulfonic acid pH 2.0 / acetonitrile (92:8) |
| Mobile Phase B | sodium octane-1-sulfonic acid pH 2.0 / acetonitrile (54:46) |
| Gradient | *Time* (min)    % Mobile Phase A    % Mobile Phase B |

| | *Time* (min) | % Mobile Phase A | % Mobile Phase B |
|---|---|---|---|
| | 0 | 100 | 0 |
| | 2 | 100 | 0 |
| | 8 | 68 | 32 |
| | 27 | 47 | 53 |
| | 32 | 0 | 100 |
| | 35 | 0 | 100 |
| | 37 | 100 | 0 |
| | 42 | 100 | 0 |

| | |
|---|---|
| Flow | 0.85 ml / min |
| Injection volume | 75 $\mu$l |
| Wavelength | 230 nm |
| Running time | Gradient - 37 min + equilibration time ~ 5 min (RT Naloxone ~ 8.3 min / RT Buprenorphine ~ 22.9 min) |

The following reagents were used: Acetonitrile HPLC quality, Methanol HPLC quality, Sodium octane-1-sulfonic acid f. ion pair chromatography, Ortho-phosphoric acid 85 % p.a., Water HPLC quality, Standard Buprenorphine, Standard Naloxone.

**Preparation of the mobile phase:**

Buffer solution:

1.1 g Sodium octane-1-sulfonic acid was dissolved in 800 ml of water while stirring vigorously. The pH value was adjusted to 2.0 with 85 % phosphoric acid. The solution was filled up to 1000 ml with water and mixed well. The pH value was checked and adjusted if necessary.
Mobile phase A: 920 ml of buffer solution and 80 ml of acetonitrile were mixed.
Mobile phase B: 540 ml of buffer solution and 460 ml of acetonitrile were mixed.

**Preparation of the solvent (LM):**

1000 ml water are adjusted to pH 2.0 with 85 % phosphoric acid.

**Preparation of the Buprenorphine / Naloxone standard solutions:**

Stock solution Naloxone:

About 20.00 mg of Naloxone standard (24.43 mg Naloxone HCl dihydrate) was accurately weighed into a 100 ml volumetric flask of amber glass. The flask was filled up to $^2/_3$ of its volume with solvent, shaken vigorously and treated in an ultrasonic bath for 5 min. After cooling down, it was filled up to the mark with solvent and the solution was mixed well. Nominal concentration of Naloxone standard was 0.20 mg/ml.

Stock solution Buprenorphine:

About 40.00 mg of Buprenorphine standard (43.12 mg Buprenorphine HCl) was accurately weighed into a 50 ml volumetric flask of amber glass. After adding 25 ml methanol to the flask, it was shaken vigorously and treated in an ultrasonic bath for 5 min. The flask was filled up to $^2/_3$ of its volume with solvent, then it was slightly swirled and filled up to the mark with solvent. The solution was mixed well. Nominal concentration of Buprenorphine standard was 0.80 mg/ml.

**Preparation of the sample solution for the test on impurities:**

2.5 ml methanol was added to the volumetric flask and it was shaken for 5 min on a mechanical shaker with minimum 200 rpm. Afterwards the volumetric flask was filled up to approx. 2/3 of its volume with solvent and shaken for another 10 min on a mechanical shaker with minimum 200 rpm. Then the volumetric flask was filled up to the mark with solvent, the solution was mixed well and filtered through a disposable filter of 0.45 μm with light brown rim, discarding the first 3 ml. 5.0 ml of each preparation are thoroughly mixed in an appropriate vessel and the mixture was injected once.

**Measurement of Retention time / Relative Retention:**

1.0 ml of each standard stock solutions were mixed with 1.0 ml of sample solution and 75 μl of each mixture was injected afterwards.

The UV spectra between 200 and 400 nm of a sample solution and the corresponding standard stock solution for Naloxone and for Buprenorphine were recorded.

| Sample | Retention time [min] | Relative retention (referring to Naloxone) | Relative retention (referring to Buprenorphine) |
|---|---|---|---|
| Solvent | no significant peaks | -- | -- |
| Eluent | no significant peaks | -- | -- |
| Naloxone-10α-Hydroxy | ~ 6.0 | 0.72 | -- |
| Naloxone-N-Desallyl | ~ 6.5 | 0.78 | -- |
| Naloxone | ~ 8.3 | 1.00 | -- |
| Buprenorphine | ~ 22.9 | -- | 1.00 |
| Buprenorphine-Dimer | ~ 32.7 | -- | 1.43 |

The relative retension time was calculated by the following formula

$$\text{Relative retention time} = \frac{RT_{(VU)}}{RT_{(WS)}}$$

$RT_{(VU)}$ = *Retention time of the impurity (in min)*

$RT_{(WS)}$ *Retention time of the active substance (in min)*

The area of the impurity of buprenorphine dimer contained in the sample was divided by 3.4.

Analyzed impurities consisted in particular of naloxone HCl derivate naloxone-10a-hydroxy and naloxone-N-desalkyl as well as of buprenorphine HCl derivate buprenorphine-descyclopropylmethyl and buprenorphine-dimer.

Table 3 discloses the percentages of impurities found in the film composition of Example 1 over storage time from 0 to 2 months.

Table 3 Total amount of impurities greater than reporting level over storage time - comparison between invention composition and prior art (AS II = accelerated stability testing conditions: T = 40 °C, RH = 75 %; PS = Permanent stability testing conditions: T = 25 °C, RH = 60 %; M = month(s), RL = reporting level)

|  | Invention composition | | | | | | Prior art composition | | | | | |
|---|---|---|---|---|---|---|---|---|---|---|---|---|
| Storage time | 0 M | | 1 M | | 2M | | 0 M | | 1 M | | 2M | |
| Stability testing conditions | PS | ASII | PS | ASII | PS | ASII | PS | ASII | PS | ASII | PS | ASII |
| Total amount of impurities > RL | 0.15 | - | 0.15 | 0.67 | 0.34 | 0.71 | 3.66 | - | 3.97 | 4.82 | 3.92 | 5.05 |

Results revealed a total amount of impurities over reporting level of between 0.15 % and 0.71 %. Standard prior art compositions comprising artificial flavoring agents or mixtures of natural and artificial flavoring agents revealed higher amount of impurities in the range of 3.66 % to 6.12 % over reporting level when stored under identical conditions and samples at identical time points.

The aspects, advantageous features and preferred embodiments of the present invention summarized in the following items, respectively alone or in combination, further contribute to solving the object of the invention:

1. An orodispersible film composition comprising:

a therapeutically effective amount of at least one active pharmaceutical ingredient,
a gelling agent,
at least one flavoring agent selected from natural citrus fruit derived oil or a mixture of two or more natural citrus fruit derived oils, wherein said composition does not comprise an artificial flavoring agent.

2. The orodispersible film composition according to item 1, wherein the active pharmaceutical ingredient is selected from the group consisting of opioids, opioid antagonists and combinations thereof, preferably is selected from buprenorphine and naloxone or pharmaceutically acceptable salts or combinations thereof.

3. The orodispersible film composition according to any one of item 1 or 2, wherein the active pharmaceutical ingredient a combination of buprenorphine HCl and naloxone HCl.

4. The orodispersible film composition according to item 3, wherein a ratio of buprenorphine HCl and naloxone HCl in the film composition of between 1:1 and 10:1, preferably between 2:1 and 5:1, more preferably 4:1.

5. The orodispersible film composition according to any one of the preceding items, wherein a percentage of the at least one active pharmaceutical ingredient is between 1 % and 50 % by weight (w/w), preferably 5 % to 25 % (w/w), more preferably 20 % (w/w).

6. The orodispersible film composition according to any one of the preceding items, wherein a percentage of flavoring agent is between 0.01 % and 1 % by weight (w/w), preferably between 0.05 % and 0.5 % (w/w), more preferably 0.1 % (w/w).

7. The orodispersible film composition according to any one of the preceding items, wherein the citrus fruit derived natural oil is selected from the group consisting of lemon, orange and grapefruit oil, or mixtures thereof.

8. The orodispersible film composition according to any one of the preceding items, wherein a percentage of the gelling agent is between 20 % and 60 % by weight (w/w), preferably 50 % (w/w).

9. The orodispersible film composition according to any one of the preceding items, wherein the gelling agent is selected from the group consisting of hydroxypropylcellulose, hydroxypropylmethylcellulose, methylcellulose, polyethylene glycol, polyethylene oxide, ethylcellulose and mixtures thereof.

10. The orodispersible film composition according to any preceding items further comprising at least one plasticizer, at least one sweetener, at least one buffering agent and/or at least one dye.

11. The orodispersible film composition according to item 10, wherein the plasticizer is selected from the group consisting of glycerol, propylene glycol, dibutyl sebacate, triacetin, triethyl citrate and isopropyl myristate and mixtures thereof, preferably propylene glycol, and/or
the sweetener is selected from the group consisting of monosaccharides, disaccharides, polysaccharides, especially maltodextrin, sucralose, neotame, alitame, cyclamate, sorbitol, xylitol, saccharin, aspartame, acesulfame, maltitol or mixtures thereof and/or the buffering agent is selected from the group consisting of tri-sodium citrate and citric acid monohydrate, or mixtures thereof.

12. The orodispersible film composition according to any one of preceding items, wherein

- a percentage of the at least one active pharmaceutical ingredient is between 1 % and 50 % by weight (w/w), preferably between 5 % and 25 % (w/w), more preferably 20 % (w/w);
- a percentage of flavoring agent is between 0.01 % and 1 % by weight (w/w), preferably between 0.05 % and 0.5 % (w/w), more preferably 0.1 % (w/w); and
- a percentage of the gelling agent is between 20 % and 60 % by weight (w/w), preferably 50 % (w/w).

13. The orodispersible film composition according to any one of preceding items, wherein

- a percentage of the plasticizer is between 2 % and 15 % by weight (w/w), preferably 5 % (w/w),
- a percentage of the sweetener is between 0 % and 20 % by weight (w/w), preferably 15 % (w/w), and/or
- a percentage of the dye is between 0 % and 0.5 % by weight (w/w), preferably between 0.001 % and 0.1 % (w/w), more preferably 0.01 % (w/w).

14. The orodispersible film composition according to any one of the preceding items, comprising

- a therapeutically effective amount of at least one active pharmaceutical ingredient,
- a plasticizer,
- a buffer substance,
- a sweetener,
- a film former,
- a dye, and
- at least one natural oil.

15. The orodispersible film composition according to any one of the preceding items, consisting of

- a therapeutically effective amount of a first active pharmaceutical ingredient, in particular buprenorphine HCl,
- a therapeutically effective amount of a second active pharmaceutical ingredient, in particular naloxone HCl,
- a plasticizer, in particular propylene glycol
- a first buffer substance, in particular tri-sodium citrate,
- a second buffer substance, in particular citric acid monohydrate,
- a first sweetener, in particular acesulfame K,
- a second sweetener, especially maltitol,
- a first film former, in particular polyethylene oxide 100,000
- a second film former, in particular polyethylene oxide 200,000
- a third film formers, in particular polyethylene oxide 900,000
- a fourth film former, in particular Pharmacoat 606,
- a dye, in particular dye FD & C Yellow No. 6, and
- a natural oil, in particular natural lemon oil.

16. The orodispersible film composition according to item 15, wherein

- a percentage of the first active pharmaceutical ingredient, preferably buprenorphine HCl is from 3 % to 20 % by weight (w/w), more preferably from 4 % to 20 % (w/w), most preferably from 4.6 % to 18.4 % (w/w),
- a percentage of the second active pharmaceutical ingredient, preferably naloxone HCl is from 0.5 % to 6 % (w/w), more preferably from 1 % to 5 % (w/w), most preferably from 1.19% to 4.8 % (w/w)
- a percentage of the plasticizer, preferably of propylene glycol is from 1 % to 4 % (w/w), more preferably 2.8 % (w/w),
- a percentage of the first buffer substance, preferably of tri-sodium citrate is from 1 % to 4 % (w/w), more preferably 2.9 % (w/w),
- a percentage of the second buffer substance, preferably of citric acid monohydrate is 2 % to 8 % (w/w), more preferably 6.3 % (w/w),
- a percentage of the first sweetener, preferably of acesulfame K is 1 % to 4 % (w/w), more preferably 3.2 % (w/w),
- a percentage of the second sweetener, preferably of maltitol is 5 % to 15 % (w/w), more preferably 12.9 % (w/w),

- a percentage of the first film former, preferably of polyethylene oxide 100,000 is 8 % to 14 % (w/w), more preferably 12.9 % (w/w),
- a percentage of the second film former, preferably of polyethylene oxide 200,000 is 20 % to 30% (w/w), more preferably 28.9 % (w/w),
- a percentage of the third film former, of polyethylene 900,000 is 1 % to 6 % (w/w), preferably 5.1 % (w/w),
- a percentage of the fourth film former, preferably of Pharmacoat 606 is 3 % to 10 % (w/w), more preferably 4.5 % (w/w),
- a percentage of the dye, preferably dye FD & C Yellow No. 6 is 0.01 % to 0.5 % (w/w), more preferably 0.03 % (w/w), and
- a percentage of the natural oil, preferably lemon oil is 0.01 % to 1 % (w/w), more preferably 0.1 % (w/w).

17. The orodispersible film composition according to any one of the preceding items, wherein the film is a non-mucoadhesive film, preferably a non-mucoadhesive film for sublingual administration.

18. The orodispersible film composition according to any one of items 2 to 17, wherein the content of impurities of naloxone derivatives and/or buprenorphine derivatives is less than 1 % by weight (w/w).

19. The orodispersible film composition according to item 18, wherein the content of impurities of naloxone derivatives, in particular naloxone-10a-hydroxy and/or naloxone-N-desalyl is less than 1% (w/w).

20. The orodispersible film composition according to item 18, wherein the content of impurities of buprenorphine derivatives, in particular buprenorphine-descyclopropylmethyl and/or buprenorphine-dimer is less than 1% (w/w).

21. A method of preparing a pharmaceutical film composition as defined by any of the preceding items, comprising the steps of:

(i) forming a suspension of a therapeutically effective amount of at least one active pharmaceutical ingredient,
(ii) adding a gelling agent to the suspension,
(iii) adding a natural oil, derived from citrus fruits, in particular lemon, orange or grapefruit oil or mixtures thereof to the suspension,
(iv) forming a film and
(v) drying the film.

22. The method of item 21, wherein step (v) is carried out at 20 to 80 degrees Celsius, preferably at 30 to 70 degrees Celsius, more preferably at 40 to 65 degrees Celsius, most preferably at less than 50 degrees Celsius.

23. The method according to item 21 or 22, wherein a drying time of between 1 and 60 minutes, preferably between 5 and 45 minutes, in particular 10 minutes.

24. The method according to any one of items 21 to 23, wherein
a drying time of between 1 and 60 minutes, preferably between 5 and 45 minutes, in particular 10 minutes at 35 degrees Celsius,
a drying time of between 1 and 60 minutes, preferably between 5 and 45 minutes, in
particular 10 minutes at 45 degrees Celsius,
a drying time of between 1 and 60 minutes, preferably between 5 and 45 minutes, in particular 10 minutes at 55 degrees Celsius, and/or
a drying time of between 1 and 60 minutes, preferably between 5 and 45 minutes, in particular 10 minutes at 65 degrees Celsius.

25. The method according to any one of items 21 to 24, wherein in step (iii) further substance selected from the group consisting of sweetening agent, plasticizers, buffering agent and/or dye is added to the suspension.

26. The method of item 25, wherein the further substance(s) being added to the suspension before, after or simultaneously with the at least one active pharmaceutical ingredient, the gelling agent and/or the natural oil.

27. The method according to any one of items 21 to 26, wherein the film is formed by casting, molding, extrusion or spraying.

28. The method according to any one of items 21 to 27, wherein the wet film has the thickness of the film is 400 to 800 microns, preferably of 700 to 750 microns, more preferably 460 to 490 microns and/or a dry film has the thickness of 100 to 200 microns, preferably of 140 to 180 microns.

29. An orodispersible film having a composition according to any one of items 1 to 20, and/or prepared by a method according to any of items 21 to 28.

30. Use of the orodispersible film composition according to any one of items 1 to 20 or the orodispersible film according to item 29 as a medicament.

**Claims**

1.  An orodispersible film composition comprising
    a therapeutically effective amount of at least one active pharmaceutical ingredient,
    a gelling agent,
    at least one flavoring agent selected from natural citrus fruit derived oil or a mixture of two or more natural citrus fruit derived oils, wherein said composition does not comprise an artificial flavoring agent.

2.  The orodispersible film composition according to claim 1, wherein the active pharmaceutical ingredient is selected from the group consisting of opioids, opioid antagonists and combinations thereof, preferably is selected from buprenorphine and naloxone or pharmaceutically acceptable salts or combinations thereof.

3.  The orodispersible film composition according to claim 1 or 2, wherein the active pharmaceutical ingredient is a combination of buprenorphine HCl and naloxone HCl.

4.  The orodispersible film composition according to claim 3, wherein a ratio of buprenorphine HCl and naloxone HCl in the film composition is between 1:1 and 10:1, preferably between 2:1 and 5:1, more preferably 4:1.

5.  The orodispersible film composition according to any one of the preceding claims, wherein a percentage of the at least one active pharmaceutical ingredient is between 1 % and 50 % by weight (w/w), preferably 5 % to 25 % (w/w), more preferably 20 % (w/w).

6.  The orodispersible film composition according to any one of the preceding claims, wherein a percentage of flavoring agent is between 0.01 % and 1 % by weight (w/w), preferably between 0.05 % and 0.5 % (w/w), more preferably 0.1 % (w/w).

7.  The orodispersible film composition according to any of the preceding claims, wherein the citrus fruit derived natural oil is selected from the group consisting of lemon, orange and grapefruit oil, or mixtures thereof.

8.  The orodispersible film composition according to any one of the preceding claims, wherein a percentage of the gelling agent is between 20 % and 60 % by weight (w/w), preferably is 50 % (w/w) and/or wherein the gelling agent is selected from the group consisting of hydroxypropylcellulose, hydroxypropylmethylcellulose, methylcellulose, polyethylene glycol, polyethylene oxide, ethylcellulose and mixtures thereof.

9.  The orodispersible film composition according to any one of preceding claims further comprising at least one plasticizer, at least one sweetener, at least one buffering agent and/or at least one dye.

10. The orodispersible film composition according to any one of claims 2 to 9, wherein the content of impurities of naloxone derivatives and/or buprenorphine derivatives is less than 1 % by weight (w/w).

11. A method of preparing a pharmaceutical film composition according to any of the preceding claims, comprising the steps of:

    (i) forming a suspension of a therapeutically effective amount of at least one active pharmaceutical ingredient,
    (ii) adding a gelling agent to the suspension,
    (iii) adding a natural oil, derived from citrus fruits, in particular lemon, orange or grapefruit oil or mixtures thereof to the suspension,

(iv) forming a film and
(v) drying the film.

12. The method of claim 11, wherein step (v) is carried out at from 20 to 80 degrees Celsius, preferably at from 30 to 70 degrees Celsius, more preferably at from 40 to 65 degrees Celsius, most preferably at less than 50 degrees Celsius.

13. The method according to claim 11 or 12, wherein a drying time is between 1 and 60 minutes, preferably between 5 and 45 minutes, more preferably 10 minutes.

14. An orodispersible film having a composition according to any one of claims 1 to 10, and/or prepared by a method according to any of claims 11 to 13.

15. Use of the orodispersible film composition according to any one of claims 1 to 10 or the orodispersible film according to claim 14 as a medicament.

Europäisches
Patentamt

European
Patent Office

Office européen
des brevets

# EUROPEAN SEARCH REPORT

Application Number

EP 12 00 7710

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 2011/017483 A2 (RECKITT BENCKISER HEALTHCARE [GB]; MYERS GARRY L [US]; HILBERT SAMUEL) 10 February 2011 (2011-02-10) | 1-5,7-15 | INV. A61K9/70 A61K47/44 A61K31/485 |
| A | * the whole document * | 6 | |
| X | EP 1 752 127 A1 (KYUKYU YAKUHIN KOGYO KK [JP]) 14 February 2007 (2007-02-14) * paragraph [0103] - paragraph [0114] * | 1,5-9, 14,15 | |
| X | US 2003/211136 A1 (KULKARNI NEEMA [US] ET AL) 13 November 2003 (2003-11-13) * claims 1, 17, 18 * * examples * * paragraph [0016] * * paragraph [0031] * * paragraph [0047] * * paragraph [0048] * * paragraph [0049] * * paragraph [0056] * | 1,2,5-9, 11-15 | |
| A | SINGH P ET AL: "Chemical profile, antifungal, antiaflatoxigenic and antioxidant activity of Citrus maxima Burm. and Citrus sinensis (L.) Osbeck essential oils and their cyclic monoterpene, dl-limonene", FOOD AND CHEMICAL TOXICOLOGY, PERGAMON, GB, vol. 48, no. 6, 1 June 2010 (2010-06-01), pages 1734-1740, XP027400140, ISSN: 0278-6915 [retrieved on 2010-04-09] * page 1735, left-hand column, paragraph 1 * * page 1737, right-hand column, last paragraph * * page 1739, left-hand column, paragraph 3 - right-hand column, paragraph 1 * | 4,6 | TECHNICAL FIELDS SEARCHED (IPC) A61K |

The present search report has been drawn up for all claims

| Place of search | Date of completion of the search | Examiner |
|---|---|---|
| The Hague | 12 April 2013 | Marttin, Emmeline |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

                      

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 12 00 7710

This annex lists the patent family members relating to the patent documents cited in the above-mentioned European search report.
The members are as contained in the European Patent Office EDP file on
The European Patent Office is in no way liable for these particulars which are merely given for the purpose of information.

12-04-2013

| Patent document cited in search report | | Publication date | Patent family member(s) | | Publication date |
|---|---|---|---|---|---|
| WO 2011017483 | A2 | 10-02-2011 | AR | 078417 A1 | 09-11-2011 |
| | | | AU | 2010279440 A1 | 29-03-2012 |
| | | | CA | 2770180 A1 | 10-02-2011 |
| | | | CN | 102548535 A | 04-07-2012 |
| | | | CO | 6511219 A2 | 31-08-2012 |
| | | | EP | 2461795 A2 | 13-06-2012 |
| | | | JP | 2013501717 A | 17-01-2013 |
| | | | KR | 20120059538 A | 08-06-2012 |
| | | | PE | 11362012 A1 | 30-08-2012 |
| | | | SG | 178265 A1 | 29-03-2012 |
| | | | US | 2011033541 A1 | 10-02-2011 |
| | | | WO | 2011017483 A2 | 10-02-2011 |
| EP 1752127 | A1 | 14-02-2007 | EP | 1752127 A1 | 14-02-2007 |
| | | | JP | 4547994 B2 | 22-09-2010 |
| | | | JP | 2005342154 A | 15-12-2005 |
| | | | US | 2007237871 A1 | 11-10-2007 |
| | | | US | 2013064874 A1 | 14-03-2013 |
| | | | WO | 2005117803 A1 | 15-12-2005 |
| US 2003211136 | A1 | 13-11-2003 | AR | 044077 A1 | 24-08-2005 |
| | | | AU | 2004233737 A1 | 11-11-2004 |
| | | | BR | PI0409715 A | 02-05-2006 |
| | | | CA | 2521735 A1 | 11-11-2004 |
| | | | CL | 8372004 A1 | 18-03-2005 |
| | | | CN | 1809343 A | 26-07-2006 |
| | | | EP | 1635796 A1 | 22-03-2006 |
| | | | GT | 200400080 A | 03-03-2005 |
| | | | JP | 2006524675 A | 02-11-2006 |
| | | | MX | PA05011508 A | 15-12-2005 |
| | | | PA | 8601401 A1 | 28-03-2005 |
| | | | PE | 03792005 A1 | 17-05-2005 |
| | | | US | 2003211136 A1 | 13-11-2003 |
| | | | US | 2008020024 A1 | 24-01-2008 |
| | | | UY | 28285 A1 | 30-11-2004 |
| | | | WO | 2004096192 A1 | 11-11-2004 |
| | | | ZA | 200508118 A | 31-01-2007 |

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2011017483 A